# EUROPEAN PATENT SPECIFICATION

(11) **EP 2 077 869 B1**
(45) Date of publication and mention of the grant of the patent: **25.02.2015**
(21) Application number: 07793964.3
(22) Date of filing: 28.08.2007
(51) Int. Cl.: A61M 1/36

(54) **A SYSTEM FOR THE EXTRA-CORPOREAL PURIFICATION OF BLOOD OF PATHOGENIC ENZYMES**
SYSTEM ZUR EXTRAKORPORALEN REINIGUNG VON BLUT VON PATHOGENEN ENZYMEN
SYSTÈME D'ÉPURATION EXTRACORPORELLE DU SANG PAR ÉLIMINATION D'ENZYMES PATHOGÈNES

(30) Priority: 28.08.2006 PL 38050006; 18.06.2007 PL 38267507
(43) Date of publication of application: 15.07.2009
(73) Proprietor: Akademia Medyczna Im. Piastow Slaskich We Wroclaw iu, 50-367 Wroclaw (PL)
(72) Inventor: SIEWINSKI, Maciej, PL-53 - 134 Wroclaw (PL); BRYJAK, Marek, PL-55 - 030 Stary Sleszow (PL); SEBZDA, Tadeusz, PL-53-208 Wroclaw (PL); KILAR, Ewa, PL-58-100 Swidnica (PL); CALKOSINSKI, Ireneusz, PL-52-129 Wroclaw (PL); JANOCHA, Anna, PL-51-109 Wroclaw (PL); KUMAR ANIL, Agrawal, PL-54-618 Wroclaw (PL); CHOROSZY- KROL, Irena, PL-56-400 Olesnica (PL); PIETKIEWICZ, Aleksander, PL-53-134 Wroclaw (PL); GRYBOS, Marian, PL-51-649 Wroclaw (PL); TRZISZKA, Tadeusz, PL-55-100 Trzebnica (PL)
(74) Representative: Witek, Rafal
(86) International application number: PCT/PL2007/000060
(87) International publication number: WO 2008/026953

(56) References cited:
- WO-A-01/17537
- WO-A-01/43770
- DE-A1- 2 532 883
- US-A- 5 725 768

## Description

The invention concerns an extra-corporeal system to purify blood of pathogenic enzymes which initiate tumoral processes as well as evoke dystrophic muscular lesion, including enzymes of the group of cysteine peptidases, in particular of cathepsins B and L and calpain, this system being intended for removing these proteins from the blood in order to eliminate the degradation of the body's cells caused by the presence of these proteins in the blood.

Information about the crucial roles of cathepsins B and L in lesions accompanying tumoral processes and of calpain in muscular dystrophic diseases is known from the latest reports in the literature. Both of these groups of enzymes belong to the family of cysteine peptidases, whose activity is inhibited by inhibitors of similar structure. These enzymes play key roles in the development of tumors and dystrophic lesions and also arise due to the presence of microorganisms in the blood which accompanies the development of inflammation in the blood, defined as sepsis. This reaction is a response of the organism to the activity of an array of destructive factors which, if unchecked due to loss of apoptotic mechanisms, results in the development of systemic inflammatory response syndrome (SIRS), manifested by multi-organ dysfunction syndrome (MODS). The elimination of enzymes from the group of cysteine peptidases, including calpain and cathepsins B and L, from the blood should prevent excessive destructive impact of these enzymes on organ cells and endothelium, causing an increase in their permeability as well as escalated apoptosis. It is probable that eliminating these enzymes may lead to decreased lysis of normal tissue, which may ultimately lead to limiting the spread of inflammation in the organism (SIRS) and thus protect the functioning of the circulatory, respiratory, immunological, and other systems.

According to publications by E. A. Auserwald et al. (1996, Eur. J. Biochem 235, 534-542) and M. Abrahamson et al. (2003, Biochem. Soc. Symp. 179-199), one can decrease, and even inhibit, the level of these enzymes in a patient's blood with the aid of specific cysteine peptidase inhibitors obtained from cancer patients' urine, albumin, placenta, and amniotic fluid as well as from soy beans, rice, and potatoes which show such low toxicity to organisms that they present the possibility of their application in inhibiting cysteine peptidases *in vivo.*

The cysteine peptidase inhibitors known from Polish patents nos. 166834, 174636, and 190404 are among such inhibitors. Cysteine peptidase inhibitors from albumin, placenta, and amniotic fluid were used in experimental animals inoculated with human tumor cells and then subjected to photodynamic therapy (Y. Saleh et al., In Vivo 2001: 15, 351-357). These studies were to investigate a modified method of malignant tumor therapy. The inhibitors were also applied in another modified method of tumor inhibition, administering them together with vitamin E (T. Sebzda et al. World J. Gastroenterol. 2005: 11, 587-592).

One of the known methods of purifying blood extra-corporeally is dialysis. The first dialyzer was constructed in 1913 and patented in 1942 in the United States. It was used in the construction of an artificial kidney, an apparatus intended to dialyze the blood of patients with renal failure (W. J. Kolff, US patent no. 5487827). The purpose of an artificial kidney is to replace kidney function, i.e. the filtering of blood, which ultimately leads to removing harmful metabolic products from it, mainly urea and creatinine. Various diseases lead to renal failure, and using a hemodialyzer assures restoring to the patient correct, periodic organic function which was disturbed by the increased blood levels of urea and creatinine.

The most important part of the artificial kidney is a container, the dialyzer, in which there is usually room for 50 ml of blood. The rinsing of excess urea and creatinine from the blood takes place in the dialyzer. The frequency of dialysis is not predetermined, but chosen for each patient individually. A dialysis session ends when the level of compounds rinsed out of the blood reaches a level allowing correct functioning of both the kidneys and other organs. This process takes place continuously and is stopped when the correct level of isolated compounds is reached. The frequency of dialysis sessions depends on the speed at which creatinine and urea accumulate in the particular patient's blood, i.e. it closely depends on the dialysis-patient relation.

As known from US patent no. 5487827, the dialyzer system consists of a container with concentrated dialysis solution. The pressure outlet of a peristaltic pump is connected to a heater and to the input of the dialysis fluid. The blood input to the dialyzer is connected to an additional peristaltic pump and to an anticoagulant dispenser to inhibit blood clotting, but if clots arise despite this precaution, they are retained in the dialyzer and do not reach the blood transfused to the patient. The pressure of the blood after leaving the dialyzer but before returning to the patient's vein is also monitored.

In summary, the dialyzer is included in the circulation through a peristaltic pump connected to the patient's vein by means of a vascular catheter with a cruciform stopcock through which blood is continuously drawn, and then through an ultrasonic air detector and pressure meter, through which the purified blood returns continuously to the patient's vein through a catheter for vascular infusion with stopcock. The dialyzer, a component of the artificial kidney, contains ca. 11,000 capillary tubes 200-300 microns in diameter. These tubes are enclosed in the container into which dialysis fluid flows with the proper pH and electrolyte concentration. The capillary tubes are made of semipermeable membrane. In this way, urea and creatinine flow through in the predetermined direction, i.e. from the blood, where their concentrations are high, to the fluid, where their concentrations are low. The difficulty connected with the construction of a dialyzer lies in the necessity of removing that which is not needed from the blood while not altering its other characteristics and not removing proteins or ions necessary for the patient's survival. Water and electrolytes can move through the semipermeable membrane in both directions, thanks to which their blood concentrations remain unaltered.

The invention involves a system for the extra-corporeal purification of blood of pathogenic enzymes by means of known carriers of cysteine peptidase inhibitors, in particular those obtained from albumin.

The essence of the invention is that in the system is a column fitted with valves on both ends. The circuit consists of a terminal valve connected to a first peristaltic pump, whose delivery side is connected to a three-way valve, one of whose outlets is connected to the entry valve of the column, whose exit valve is connected to a second peristaltic pump, whose delivery side is connected to a second terminal valve. These components of the system are connected by medical-quality plastic tubes. The column is filled with granular sorbent or fiber, in particular granulated or modified cellulose or acrylic copolymers or sorbent coated with hydroxymethyl methacrylate of 300-500 micron granulation and pore-size of 0.1 to 10 microns, or fibers 200 microns in diameter, whereby the sorbent is permeated with cysteine peptidase inhibitors to a quantity of 20-100 mg/g of carrier (sorbent). Inside both ends of the column are porous supports on which rest polypropylene or polyamide porous membranes of mesh size not greater than 20 microns.

Preferentially is when the exit of a container equipped with a piston and filled with anticoagulant leads to the second entry of the three-way valve.

It is also preferentially when the inside of the column is divided into a series of segments by means of membranes, whereby in each segment are 20-50 ml of the absorbing deposit.

The basic task of the system according to the invention is to assure that only specific harmful, pathogenic enzymes are continuously removed from the patient's blood, while the concentrations of other components remain unchanged. The blood is thus freed exclusively of pathogenic muscular cysteine peptidases of the patient's own cells without the necessity of direct contact with the patient's organism.

Further, the present invention relates to the application of cysteine protease inhibitors bound to insoluble carriers in the *ex vivo* purification of blood, particularly of cathepsin B and L and calpains obtained from various sources such as chicken egg proteins, amniotic fluid or isolated from microorganisms.

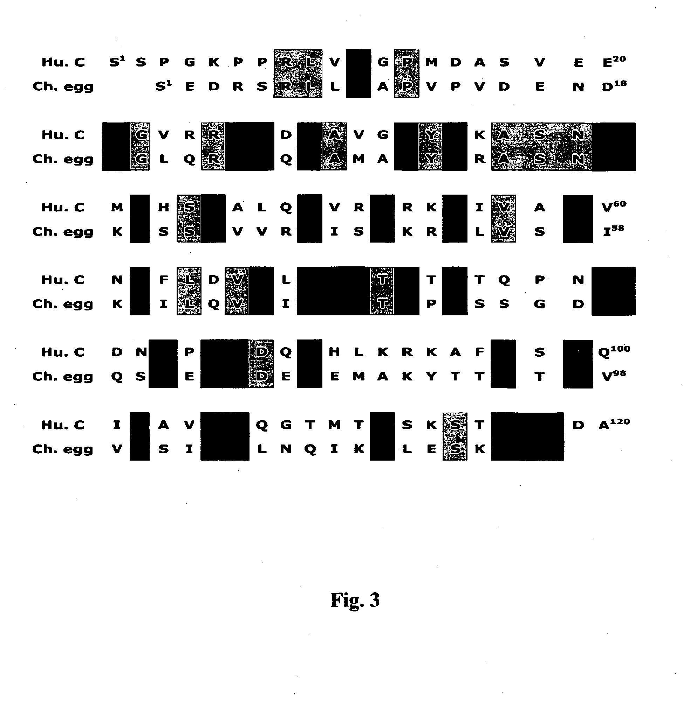

The nature of this aspect consists of collecting blood from patients which contains the aforementioned cysteine proteases. The blood is then passed for at least one cycle through membrane columns or other filters containing activated inhibitors of said cysteine proteases attached to insoluble carriers approved for use in medical implements, particularly in biopolymers, wherein in such bioabsorbents, the cysteine proteases are removed *ex vivo* via affinity chromatography and the eluate obtained - thusly purified blood - is re-introduced into the patient's blood stream or is portioned and stored using methods known from blood donation for a period no longer than two weeks.
Many "cysteine protease inhibitors" are known which may be used according to the present invention. For nearly twenty years it has been described in many reports that specific enzymes containing cysteine residues in their active centers, such as cathepsins B and L, play a key role in tumor transformation, invasion, metastases and angiogenesis (Schmitt M., Janicke F., Graeff H. (1992): Tumor-associated proteases. Fibrynolysis 6, 3 - 26). Similar suggestions have appeared following a series of studies on diseases involving muscle autolysis such as muscular dystrophy and multiple sclerosis as well as other autogenic tissue dystrophies. In the latter case, inhibitors are sought for enzymes of the calpain type, which include the peptides calpastatins including one designated DIA19 (Carragher NO. Calpain inhibition: a therapeutic strategy targeting multiple disease states Curr. Pharm. Des., 2006, 12, 615 -638). A natural response to this information was to seek specific inhibitors which could inhibit these processes. The first studies seeking specific inhibitors of these enzymes resulted in the production of effective inhibitors but only *in vitro,* because *in vivo* they were too toxic. An exception to this were microbial peptides, which despite their cost give hope for use *in vivo* (for now in animal models). The initial inhibitors to be used in *in vivo* on a larger scale were at first obtained from the urine of cancer patients (Siewisńki M. (1993): Autologous cysteine peptidase inhibitors as potential anticancer drugs. Anti - Cancer Drugs 4, 97- 99), an subsequently isolated from human and animal placentae and amniotic fluid. The breakthrough in this research came with the experiments by a Japanese group, which showed a high degree of similarity between cystatins from eggs and endogenous inhibitors from human bodily fluids(Saitoh E., Isemura S., Sanada K. (1988): Cystatin superfamily. Evidence that family II cystatin genes are evolutionary related to family III cystatin genes. Biol Chem Hoppe-Seyler 369, 191 - 197) - Fig. 3. It should be expected that chicken egg cystatins may be used in new directions in antitumor therapy. The extensive homology between egg protein cystatins and human inhibitors has been confirmed. The latter are immune agents against invasive diseases. To date, the most extensively studied models in the inhibition of cathepsin B and L activity were specific inhibitor peptides including E-64 (Premzl A, Turk V, Kos J. Intracellular proteolytic activity of cathepsin B is associated with capillary-like tube formation by endothelial cells in vitro. J. Cell Biochem. 2006; 97, 1230 -1240), obtained synthetically (Sever N, Filipic M, Brzin J, Lah TT. Effect of cysteine proteinase inhibitors on murine B16 melanoma cell invasion in vitro. Biol. Chem. 2002; 383, 839 - 143). It was determined that despite their specific inhibition of cathepsin B and L *in vitro,* it proved impossible to administer them *in vivo,* due to their high toxicity to the organism (Makioka A, Kumagai M, Kobayashi S, Takeuchi T. (2005): Entamoeba invadens: cysteine protease inhibitors block excystation and metacystic development. Exp. Parasitol.109: 27 - 32). To date, the most extensively studied models in the inhibition of cathepsin B and L activity were specific inhibitor peptides including E-64 (Premzl A, Turk V, Kos J. Intracellular proteolytic activity of cathepsin B is associated with capillary-like tube formation by endothelial cells in vitro. J. Cell Biochem. 2006; 97, 1230 -1240), obtained synthetically. It was determined that despite their specific inhibition of cathepsin B and L *in vitro,* it proved impossible to administer them *in vivo,* due to their high toxicity to the organism (Makioka A, Kumagai M, Kobayashi S, Takeuchi T. (2005): Entamoeba invadens: cysteine protease inhibitors block excystation and metacystic development. Exp. Parasitol.109: 27 - 32). It turned out that cysteine protease inhibitors obtained from the uring of cancer patients, egg proteins, placentae and amniotic fluid as well as soya, rice, potatoes and others show no toxicity to living organisms. It was showed that the inhibitors isolated from egg proteins are genetically homologous to a human inhibitor, cystatin C. Cystatins being endogenous inhibitors of cystein peptidases play a role in immunity against invasive diseases including tumors and dystrophies (Kos J, Werle B, Lah T, Brunner N. (2000): Cysteine proteinases and their inhibitors in extracellular fluids: markers for diagnosis and prognosis in cancer. Int J Biol Markers 15: 84 - 89). Cysteine inhibitors from the mentioned sources, i.e. egg proteins, have been adminisered to experimental animals *in vivo,* which had human tumors implanted in them. The results confirmed that there exists a possibility of inhibiting cysteine peptidase activity directly in patients (*in vivo*). Polish patent No. 190404 relates to an anti-tumour, anti-microbial and anti-invasive disease pharmaceutical agent whose active ingredients are cysteine protease inhibitors with molecular masses of 9,0 to 57 kDa, isolated from placentae and amniotic fluid of humans or animals. A single dose of this agent contains 10 - 50 inhibition units at 1.0x10-4 do 1.0x10-5 mg protein/ml pharmacological carrier, adapted to a particular form of therapy, particularly in the form of an aqueous solution or oil suspension (Siewiński M. (1991): A method of obtaining thiol protease inhibitors from urine. Patent of the Academy of Medicine. 288769.; Siewiński M., Berdowska I., Jarmulowicz J. (1998): A method of obtaining cysteine peptidase inhibitors from plants and egg proteins. Patent of the Academy of Medicine PL. 174636 B.; Siewiński M., Saleh Y., Gamian A., Wnukiewicz J.(2005): A pharmaceutical agent against tumors, pathogenic microorganisms and invasive diseases (Polish patent application P.334758). These inhibitors may be administered against tumors, including cancers of the breast, liver, prostate, pancreas, gastrointestinal tract and muscular dystrophy in the form of an injection. This agent may also be administered as an aerosol for treatment of the lungs, alveoli or larynx. In a number of tumor transformations it may be administered as an injection directly into a localised tumor or as a cream or gel for topical application for skin cancer or post-radiotherapy changes. Whereas Polish patents 174636 and 332705 describe a method of obtaining cysteine protease inhibitors from egg protein via affinity chromatography. These inhibitors have molecular masses from a few to a dozen or so kilodaltons. Polish patent No. 166834 describes a method of obtaining similar inhibitors from urine. Cysteine protease inhibitors from egg protein, the placenta, and amniotic fluid were used in experimental animals with implanted human tumors, which were then treated photodynamically (Saleh Y., Ziólkowski P, Siewiński M., Milach J., Marszalik P., Rybka J. (2001): The combined treatment of transplantable solid mammary carcinona in wistar rats by use photodynamic therapy and cysteine proteinase inhibitors. In vivo, 15; 351 - 357.), or with vitamin E (Sebzda T., Saleh Y., Siewiński M., Rudnicki J., Ziólkowski P. (2005): The influence of vitamin E and human placenta cysteine peptidase inhibitor on the expression of cathepsin B and L implanted hepatoma Morris 5123 tumor model in the Wistar rats. World Journal of Gastroenterology 11, 587 - 592) in the design of experimental treatments. It should be remembered that the possible mechanism of inhibition of endogenous cysteine proteases in the human has been elucidated, using cysteine peptidase inhibitors from the urine of malignant tumor patients (Mikulewicz W., Berdowska I., Jarmulowicz J., Siewiński M. (1993): Decrease in vivo of cysteine endopeptidases in blood of patients with tumor of larynx. Anti - Cancer Drugs 4, 341 - 344.)

In the system of the present invention, all "cysteine protease inhibitors" described or suggested in the above cited reports may be used.

US 5725 768 discloses a system according to the preamble of claim 1.

Preferentially, the activity of cysteine proteases is assessed during the *ex vivo* purification of blood and on this basis the time of contact between the blood and bioabsorbent as well as the number of cycles are calculated.

Preferentially, the blood purification cycles are performed until such a time that less than 20% of the initial cysteine peptidase, activity remains in the patient's blood.

Preferentially the biosorbents containing the cysteine protease inhibitors are exchanged at pre-determined intervals following saturation with cysteine proteases. The *ex vivo* purification of blood according to the present invention may use known techniques such as plasmophoresis or hemoperfusion.
Plasmophoresis is therapeutic plasma exchange (TPE). It consists of the separation of plasma along with possible pathogens from blood morphotic elements. The removed plasma is replaced with a substituent fluid whose constituents include, in accordance with the clinical situation: homologous plasma, albumins, electrolyte solutions and colloids. The present state of knowledge of plasmophoresis makes it possible to perform it via sedimentation, centrifugation (continuous and discontinuous), filtration or the cascade method.
The sedimentation method is based on the autologous precipitation of erythrocytes following 15 - 30 minutes of maintenance of the exsanguinated blood on a haematological medium, often with the addition of a rinsing agent. The accreted plasma is removed from above the erythrocyte layer. Many variants of this technique are known, but the basis is always the same.

Discontinuous centrifugation consists of using a Janetzki centrifuge with 7-10 minut spin times at 2-3 KRPM. The separation of plasma is more stringent than in the previous method and its similicity and low cost have made it the most popular form of plasmophoresis , with various modifications. It requires vascular access in the form of one needle, and the extraorganismal blood volume in circulation is 125-375 ml which makes this method unsuitable for use in small children. Whereas during the continuous method, two needles are required, and the blood volume outside the organism is about 80 ml. The length of the continuous procedure is also much smaller.
Both of the illustrated methods are characterised by the possibility of infecting the transferred erythrocyte mass, as well as by the possibility of mechanical to the erythrocytes damage and/or haemolysis. The third method largely removes both hindrances.
MPS, membrane plasma separation, makes use of special filters in a closed arterial-venuous or venuous-venuous system, which forces the use of additional pumps to force blood flow or mechanical pressure controllers in the system, sometimes temperature stabilizers, as well as heparin treatment each time.

The construction of all modern plasmophoretic filters is similar, despite manufacture by a dozen or so different companies. The usual materials are polypropylene capillaries with a diamter of 0.5-0.6 mm and an active surface of 0.1 - 0.4 m². this makes it possible to remove elements with diamaters smaller than the capillary diamater such as immunoglobulins, lipoproteins, albumins or fibrinogen. The biggest problem of this method is the use of the correct transmembrane pressure (TMP), whose magnitude oscillates between 0,08 and 0,107 bar (60 and 80 mmHg) depending on the filter used. Too steep a pressure gradient causes erythrocyte haemolysis and as a result clogs the filter system. Thus, modern filter sets facilitate TMP control with a high degree of precision. A number of plasmophoretic pumps in the combined egress pathway also transfers the plasma substituent. This, however, may also be performed via an alternate venuous pathway and peristaltic perfusion pumps.
Like in all techniques using extravascular blood circulation, this method also requires the thermal compensation using insulation or electronic isothermal control.
In capillary dialysers, the blood flow should exceed 50ml/min, whereas the optimal flow is usually around 100-150 ml/min.
The cascade method: the development of biotechnology has made it possible to supplement the filtration method with a subsequent filtration of the separated plasma through filters of special construction, which specifically remove the potentially pathogenic plasma element while its remainder is returned into circulation. The chief advantage is the maintenance of circulating blood volume with very limited fluid subsitution. Columns of this tyme make use of immunoabsorbence (protein A, I-02) or specific chemical reactions.
The decided leader in modern plasmophoresis is the Fresenius company, along with products from such companies as Prometheus, and the DALI, Immunosorba or Prosorba systems.
The next important aspect of plasmophoresis is vascular access. When using the centrifugation methods, a blood flow of 40-50 ml/min is required. In some cases this may be achieved using a large peripheral vein (i.e. in the elbow). Whereas vascular access to a central blood vessel or an arterial-venuous bypass commonly used in dialysis are indicated when using semi-permeable membranes, which is preferential in chronic therapy, such as for hypercholesterolemia.
Anticoagulation should also be taken into account, both during centrifugation and filtration. Unfractioned heparin is used most often, and its dosage should be adjusted individually to each patient. The monitoring of coagulation times is indicated. Fractionated heparin preparations are also available. Dostepne sa także preparaty heparyny frakcjonowanej. In Poland, the *in vivo* use of citrates is forbiddedn, which are used successfully in the West.
Complications in plasmophoresis occur in 4 to 25% of treatments, on average around 10%. Mild reactions such as rashes, parestesis, nausea, and leg muscle contractions occur in about 5% of cases. Moderately severe effects (5-10%) are decreases in arterial pressure, chest pains and arrhythmia. Fatalities occur in about 3-6 cases per 10 procedures performed. Additionally, further adverse effects occur as a result of vascular access (haematoma, peripneumonary oedema) and as a result of anticoagulant treatment.

The next technique which may be used is haemoperfusion, a method of extraorganismal removal of endogenous toxins. in this method, a patient's complete blood is sampled from a large blood vessel, made to circulate outside of the organism through a column packed with an adsorbent and returns to the patient.

The absorbent materials presently used are activated carbon granules coated with a cellulose membrane or an acryl hydrogel, or non-ionic resins such as XAD-2 or XAD-4. The coating on the activated carbon prevents clot formation and the destruction of blood morphotic elements, particularly of platelets and white cells. Vascular access and anticoagulation are performed in a similar fashion to filtration plasmophoresis.

The use of *ex vivo* blood purification ensures that only cysteine peptidases are removed, whereas the other components remain unaltered. As a result, patients' blood remains unchanged except for the binding of the pathogenic enzymes such as cathepsin B or L to the bioabsorbent, and may be used again in the organism to perform a physiological function. The most significant result of this process is the retardation or inhibition of tumor development or of dystrophic changes in muscle by cathepsin B or L and calpains.

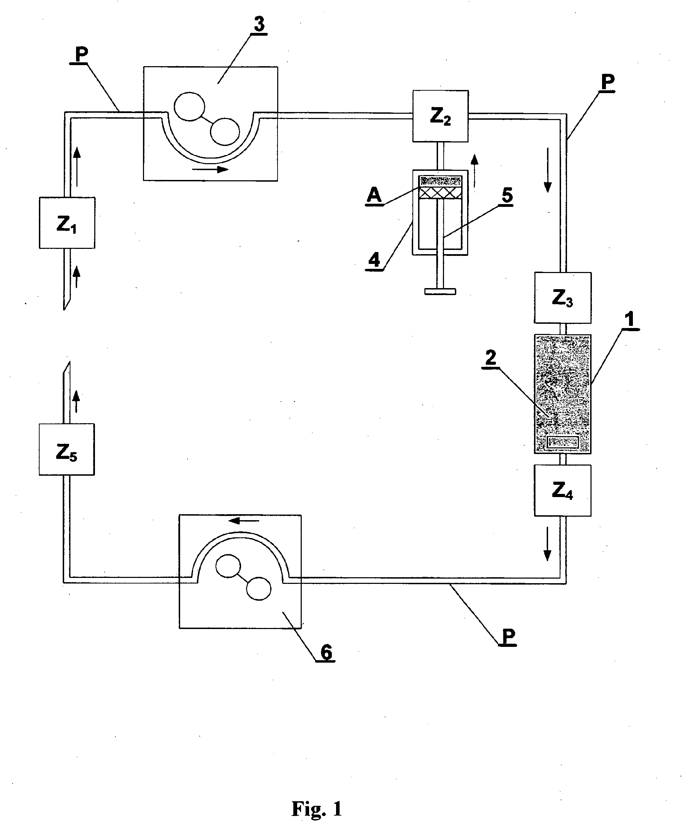

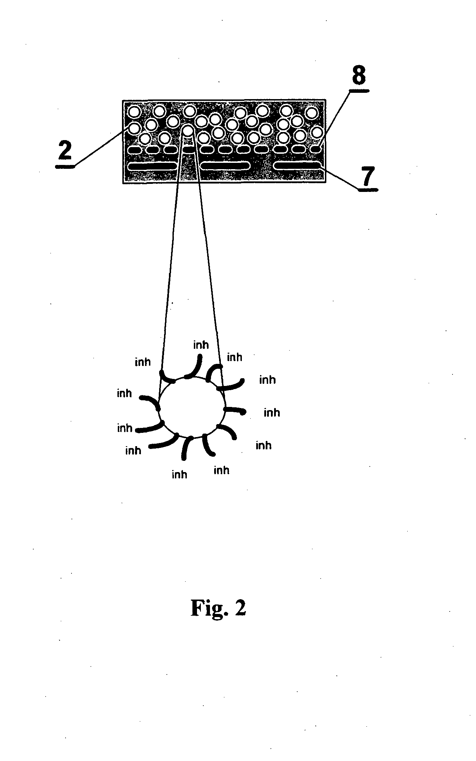

The use of a modified autotransfucion, meaning the sampling of blood from a patient, its purification and the re-introduction of the blood purified of the pathogenic enzymes into the patient resolves the problem of removing harmful complexes from the bloodstream, which are alien to the organism and may cause anaphylactic shock or prove toxic to the patient. In the proposed solution, the cysteine peptidase complexes removed via their inhibitors remain outside the patient along with the insoluble biosorbents. Thanks to this, the application of inhibitors may be used in the treatment of malignant tumors or muscular dystrophies, in which a key role is played by cysteine proteases, without the danger of toxic effects of certain cysteine peptidase inhibitors, as well as the danger of the occurrence of anaphylactic shock caused by the appearance of enzyme-inhibitor complexes in the patient.
The purification of patients' blood from cysteine peptidases occurs under conditions in which inhibitor complexes have no contact with the patient's internal environment. This form of blood purification is particularly indicated for patients for whom prospects of successful treatment with conventional means are meagre. It is justifiably predicted that the *ex vivo* inhibition of these enzymes may create a new direction in therapy, the so called extraorganismal inhibitor therapy.
It is expected that the use of the proposed ihibitor therapy may retard tumor transformation, invasions, metastases, angiogenesis or apoptosis as well as processes catalysed by calpains, which catalyse the autodestruction of muscles.
An example of the subject of the invention is presented in Fig. 1, which is a diagram of the system, and Fig. 2, showing the longitudinal section of a fragment of the column's deposit. List of labels: 1- Replaceable column, 2- Sorbent, 3- First peristaltic pump, 4- Container for anticoagulant, 5- Piston, 6- Second peristaltic pump, 7-Porous support, 8- Porous membrane, inh - Cysteine peptidase inhibitors, A - Anticoagulant, P - Tubes, Z₁ - First terminal valve, Z₂ - Three-way valve, Z₃ - Entry valve of the column, Z₄ - Exit valve of the column, Z₅ - Second terminal valve.
Figure 3 represents a comparison of the sequences of human cystatin C and a cystatin obtained from egg protein.

### Example 1. A method of immobilizing inhibitors to the surface of the material comprising the filter bed.

The method of immobilizing inhibitors on the surface of insoluble carriers (acryl polymers, cellulose and derivatives, polysaccharides or activated carbon) consists of two stages i) activation of the carrier with an appropriate agent, and subsequently ii) immobilization of the inhibitor protein via the formation of a chemical bond between it and an actiove group on the surface of the carrier. An example method of binding cystatin onto a polysaccharide is presented.
100 mL of Sepharose 4B is washed with 1 M aqueous sodium carbonate (Na2CO3). To activate carrier, 5 mL divinylosulfonate are added which are mixed with 100 mL Sepharose 4B suspension for 2 hours at room temperature. Following activation, the filter bed is washed twice with 100 mL 1 M sodium carbonate, and then thrice in 100 mL 0.1 M Na2CO3. During the final stage, the filter nbed is washed with 500 mL distilled water. The subsequent steps of washing surplus reagents are performed at room temperature, adding reagents in aliquots, mixing for 10 min and filtering onm a Buchnera funnel through a filter pad. After the last wash, the carrier is suspended in 0.1 M PBS, pH=8,5 and10 mL of inhibitor solution obtained from chicken eggs are introduced (ca. 0.5 g calculated protein). The solution is mixed at room temperature for 10-12 hours. After the immobilisation the preparation is washed with aliquots of PBS and distilled water and filtered on a Buchnera funnel until the filtrate no longer absorbs light at 280 nm. The immobilized preparation is maintained at 4°C.

### Example 2. Embodiment of the system according to the present invention

The demonstration system has a column (1) of circular cross-section, constituting a filter filled with granular sorbent (2) which is an acrylic copolymer with a granulation of 400 microns and pore-size ranging from 1 to 50 microns. The sorbent (2) is permeated with cysteine peptidase inhibitor obtained from albumin. The column (1) is in a closed circuit formed by tubes (P) made of medical-quality plastic. At the entrance of the circuit is the first terminal valve (Z₁) connected by a tube (P) to the first peristaltic pump (3), whose delivery side is connected over a three-way valve (Z₂) to the entry valve (Z₃) of the column (1) as well as to the container (4) equipped with a piston (5) and filled with anticoagulant (A). The exit valve (Z₄) of the column (1) is connected to a second peristaltic pump (6), whose delivery side is connected to the second terminal valve (Z₅). The terminal valves (Z₁ and Z₅) are adapted to connect to the vascular catheters placed in the patient's veins. Inside the entry and exit of the column (1) are porous supports (7) on which rest the porous membranes (8) made of polypropylene or polysulfone . Every granule of sorbent (2) is coated with a layer of cysteine peptidase inhibitor (inh) (25 - 100 mg inhibitor per 1 g of sorbent 2).

The system functions as follows. After connection to the patient's circulation, blood is pumped through the first terminal valve (Z₁) into column (1), where it comes in contact with the sorbent granules (2) on which inhibitor (inh) is deposited which absorbs the harmful cystein peptidases. The purified blood is pumped by the second peristaltic pump (6) back into the circulation. During purification, blood parameters are monitored, anticoagulant (A) is dispensed when needed, and the process is stopped when the cysteine peptidase activity is ca. 10% of the initial activity.
The system described above may also possess additional safeguards in the form of pressure sensors, which would alarm of the following adverse effects: improper blood supply, blood coagulation on the filter bed, as well as improper blood collection by the blood vessel. This pressure should be measured at the following points: prior to the blood pump, after the filter and between the blood pump and filter. Measured values exceeding the alarm setpoints would be indicative of problems in one of the above mentioned elements.

Additionally, it is preferential to use an air sensor in the system, protecting the patient from the ingress of air into circulation. Such a sensor would halt the blood pump upon discovery of air bubbles in the blood behind the filter or immediately prior to re-entry into the blood vessel.
The complete system would be thus (in order from entry from an aretry to egress into a vein):
a. "Arterial" pressure sensor which may indicate possible blood supply problems, cause for example by needle removal, kinked hoses, or improper catheter functioning),
b. Blood pump supplying blood onto the filter at a rate of a dozen to several hundred ml/min.
c. Pressure sensor reacting to undesirable increased blood pressure in the system which may occur in the case of blood clotting on the filter,
d. Pump supplying the anticoagulant,
e. A filter with an absorbent according to the present invention,
f. Air sensor, reacting to the presence of air in the blood system,
g. A "venuous" pressure sensor safeguarding irregularities in the reception of blood, caused for example by venuous needle egress, kinked hoses, coagulation in the air detector, undesirable blood extravascular pumping or damaged vessels and catheters).
The situation described above occurs only when the procedure may be performed on whole blood. If solely plasma is required in the procedure without morphotic elements, such a system will be different in that the plasma must first be separated so that it may be filtered. This would require the additional use of a separator and pump for the plasma itself.
In the case of each therapeutic method, systems available on the market may be used. Thus, for performing the procedure on whole blood, any haematoperfusion apparatus may be used, whereas when the procedure involves the plasma alone, one would need to use albumin dialysis or plasmophoresis systems which are capable of liquid separation of blood morphotic elements. Example systems are.: Multifiltrate (to purify whole blood) or Prometheus (plasma) from Fresenius. The whole blood procedure described may also make use of any given haemodialysis apparatus, a so-called "iron kidney".

## Claims

1. A system for extra-corporeal purification of blood from pathogenic enzymes, using known carriers of cysteine peptidases, particularly those obtained from egg protein, containing a column (1), equipped at both ends with valves ( Z₃ , Z₄ ) connected with pipes (P) of medical grade plastic, wherein the column (1) is in a closed circuit and at the entrance of the circuit is a first terminal valve ( Z₁ ) connected by a tube (P) to a first peristaltic pump (3), whose delivery side is connected over a three-way valve ( Z₂ ) to the entry valve ( Z₃ ) of the column (1), and the exit valve ( Z₄ ) of the column (1) is connected to a second peristaltic pump (6), whose delivery side is connected to a second terminal valve ( Z₅ ),
where the column (1) is filled with a granular absorbent, particularly granular or modified cellulose or acryl copolymers or absorbents coated with hydroxymethyl methacrylate with 300-500 micrometer granularity and pores in the range 0,1 - 10 micrometer, or fiber diameters of about 200 micrometer, **characterised in that** the absorbent is saturated with an inhibitor ( inh ) of cysteine peptidases at a rate of 20 to 100 mg/g of carrier, whereas at both sides, the column (1) contains internal structures (7), which support porous polyamide or polypropylene membranes (8) with pores no greater than 20 micrometers.

2. A system according to Claim 1, **characterised in that** the second entry of the three-way valve ( Z₂ ) is connected to a container (4) equipped with a piston (5) and filled with an anticoagulant (A).

3. A system according to Claim 1, **characterised in that** the interior of the column (1) is divided into a series of segments using porous membranes (7), wherein each segment contains from 20 to 50 ml of absorbent (2).

## Patentansprüche

1. System zur extrakorporalen Reinigung von Blut von pathogenen Enzymen, unter der Verwendung bekannter Träger von Cysteinpeptidasen, insbesondere denen die aus Eiprotein erhalten werden, enthaltend eine Säule (1), die an beiden Enden mit Ventilen (Z₃, Z₄) ausgerüstet ist und mit Rohren (P) aus Kunststoff medizinischen Grads verbunden ist, wobei sich die Säule (1) in einem geschlossenen Kreislauf befindet und sich am Eingang des Kreislaufs ein erstes terminales Ventil (Z₁) befindet, verbunden durch ein Rohr (P) mit einer ersten Peristaltikpumpe (3), deren Zuführungsseite über ein Dreiwege-Ventil (Z₂) mit dem Eingangsventil (Z₃) der Säule (1) verbunden ist, und das Ausgangsventil (Z₄) der Säule (1) ist mit einer zweiten Peristaltikpumpe (6) verbunden, deren Zuführungsseite mit einem zweiten terminalen Ventil (Z₅) verbunden ist,
wobei die Säule (1) mit einem granulären absorbierenden Mittel befüllt ist, insbesondere granulärer oder modifizierter Cellulose oder Acrylcopolymeren oder absorbierenden Mitteln beschichtet mit Hydroxymethylmethacrylat mit einer Granularität von 300-500 Mikrometern und Poren im Bereich von 0,1-10 Mikrometern oder Faser-Durchmessern von ungefähr 200 Mikrometern, **dadurch gekennzeichnet, dass** das absorbierende Mittel mit einem Inhibitor (inh) von Cysteinpeptidasen bei einer Rate von 20 bis 100 mg/g an Träger gesättigt ist, wobei die Säule (1) an beiden Seiten interne Strukturen (7) aufweist, die poröse Polyamid- oder Polypropylenmembranen (8) mit Poren von nicht größer als 20 Mikrometern tragen.

2. System nach Anspruch 1, **dadurch gekennzeichnet, dass** der zweite Eingang des Dreiwege-Ventils (Z₂) mit einem Behälter (4) verbunden ist, der mit einem Kolben (5) ausgestattet ist und einem antikoagulierenden Mittel (A) befüllt ist.

3. System nach Anspruch 1, **dadurch gekennzeichnet, dass** das Innere der Säule (1) unter der Verwendung von porösen Membranen (7) in eine Reihe von Segmenten aufgeteilt ist, wobei jedes Segment von 20 bis 50 ml absorbierendes Mittel (2) enthält.

## Revendications

1. Système pour la purification extracorporelle par élimination d'enzymes pathogènes, en utilisant des véhicules connus de cystéine peptidases, en particulier celles obtenues à partir d'une protéine d'oeuf, contenant une colonne (1), équipée aux deux extrémités de soupapes (Z₃, Z₄) raccordées à des tuyaux (P) de plastique de qualité médicale, dans lequel la colonne (1) est dans un circuit fermé et à l'entrée du circuit se trouve une première soupape terminale (Z₁) raccordée par un tube (P) à une première pompe péristaltique (3), dont le côté délivrance est raccordé via une soupape à trois voies (Z₂) à la soupape d'entrée (Z₃) de la colonne (1), et la soupape de sortie (Z₄) de la colonne (1) est raccordée à une deuxième pompe péristaltique (6), dont le côté délivrance est raccordé à une deuxième soupape terminale (Z₅),
où la colonne (1) est remplie d'un absorbant granuleux, en particulier des copolymères granuleux ou modifiés de cellulose ou d'acryle ou des absorbants enrobés de méthacrylate d'hydroxyméthyle ayant une granularité de 300-500 micromètres et des pores dans la plage de 0,1-10 micromètres, ou des diamètres de fibres d'environ 200 micromètres, **caractérisé en ce que** l'absorbant est saturé avec un inhibiteur (inh) des cystéine peptidases à un taux de 20 à 100 mg/g de véhicule, tandis que sur les deux côtés, la colonne (1) contient des structures internes (7), qui supportent des membranes de polyamide ou de polypropylène poreuses (8) avec des pores ne dépassant pas 20 micromètres.

2. Système selon la revendication 1, **caractérisé en ce que** la deuxième entrée de la soupape à trois voies (Z₂) est raccordée à un contenant (4) équipé d'un piston (5) et rempli d'un anticoagulant (A).

3. Système selon la revendication 1, **caractérisé en ce que** l'intérieur de la colonne (1) est divisé en une série de segments en utilisant des membranes poreuses (7), dans lequel chaque segment contient de 20 à 50 ml d'absorbant (2).
